# EUROPEAN PATENT SPECIFICATION

(11) **EP 1 052 966 B1**
(45) Date of publication and mention of the grant of the patent: **13.03.2002**
(21) Application number: 99903650.2
(22) Date of filing: 19.01.1999
(51) Int. Cl.: A61K 7/16

(54) **ORAL CARE COMPOSITIONS**
MUNDPFLEGEZUSAMMENSETZUNGEN
PRODUIT D'HYGIENE BUCCALE

(30) Priority: 05.02.1998 EP 98200343
(43) Date of publication of application: 22.11.2000
(73) Proprietor: UNILEVER N.V., 3013 AL Rotterdam (NL); UNILEVER PLC, London EC4P 4BQ (GB)
(72) Inventor: Ramos Almeida, Raquel S., Ind. Gessy Lever Ltd., CEP-05805 Sao Paulo, SP (BR)
(74) Representative: Kan, Jacob Hendrik, Dr.
(86) International application number: EP9900319
(87) International publication number: WO9939685

(56) References cited:
- WO-A-95/34275
- WO-A-96/09034
- GB-A- 2 159 412
- GB-A- 2 220 568

## Description

The present invention relates to oral care compositions in gel form. More particularly it relates to oral care compositions in transparent gel form which contain an alkalimetal bicarbonate source.

Oral care compositions in gel form are well-known in the art. They usually comprise an abrasive silica and a thickening silica in a suitable liquid vehicle comprising a polyol-humectant such as sorbitol, glycerol, lactitol, xylitol and so on, with other optional ingredients. By judicious selection of the types of abrasive silica and thickening silica, particularly as regard the refractive indices of these materials, transparent gels can be obtained, and such oral care compositions have already found their way to the market place. When including optional ingredients, however, care must be taken not to jeopardize the transparency of the gels.

Lately, oral care compositions have been developed and marketed which contain an alkalimetal bicarbonate source, e.g. sodium bicarbonate. This material can function as a mild abrasive ingredient in the oral care composition, as well as an agent to neutralize the acids, formed in the oral cavity by microbial decomposition of sugar. However the inclusion of an alkalimetal bicarbonate source in an oral care composition in gel form may jeopardize the transparency of the gel.

Currently, there is a consumer demand for oral care compositions in transparent gel form, which provide for a teeth-whitening effect as well as a reduction of oral malodour and an improved cleaning and acid-neutralising effect.

It has now surprisingly found that the inclusion of a low level of an alkalimetal bicarbonate source in an oral care composition in gel form which comprises a thickening silica and a particular abrasive silica provides for an oral care composition in gel form which is transparent, provides for a teeth-whitening effect, reduces oral malodour and neutralizes the acids, formed in the oral cavity by microbial decomposition of sugar to a significant degree. It is surprising that the inclusion of the low level of the alkalimetal bicarbonate does not jeopardize the transparency of the gel.

The alkalimetal bicarbonate source, used in the present invention can be sodium and potassium bicarbonate or sesquicarbonate or mixtures thereof, sodium bicarbonate being the preferred material. The amount of the alkalimetal bicarbonate source, included in the composition of the invention, ranges from 0.5 to 5% by weight of the composition, preferably from 0.75 to 3% by weight, and particularly preferably from 1 to 2% by weight.

The thickening silica, used in the present invention, can be any suitable thickening silica, known in the art, such as Sident 22S, Sorbosil TC15, TiXOsil 43 and Hi SiI DT 267M. The thickening silica is generally used it an amount of 3-15% preferably 5-10 by weight of the composition.

The particular abrasive silica used in the present nvention is a silica with a low refractive index. It may be used as the sole abrasive silica, or in conjunction with a low level of other abrasive silicas, e.g. those according to EP 236070.

The low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 µm, a BET (nitrogen) surface area of between 10 and 100 m2/g and an oil absorption of about 70 - 150 cm3/100 g, but abrasive silicas with a lower apparent refractive index may also be used.

Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfieid Chemicals (having an R.I. of approximately 1.440). Further examples can be found in EP-A-0,549,287 (Colgate) and in WO 94/10087, EP 535,943, EP 666,832, (Crosfield). The amount of these silicas in the composition generally ranges from 5-60 by weight, usually 5-20% by weight.

The humectants, used in the compositions of the invention are the conventional polyol-humectants such as sorbitol, glycerol, mannitol, lactitol, xylitol and mixtures thereof, usually used in an amount of 30-85% by weight, preferably 50-75% by weight. Sorbitol is the preferred humectant.

The liquid phase of the composition of the present invention should be formulated such, that its refractive index closely matches the refractive index of the silicas as discussed above. The liquid phase usually comprises water and a polyol-humectant, the latter usually being sorbitol or glycerol. Water has an R.I. of 1.333, sorbitol (70% aqueous solution) has an R.I. of 1.457 and glycerine and R.I. of 1.473. Other humectants such as propylene glycol, polypropylene glycol and polyethylene glycol may also be present. The refractive index of the liquid phase can be calculated from the respective refractive indices of the constituting ingredients.

The compositions of the invention may contain further, optional ingredients, as long as the clarity of the dentifrices is thereby not impaired and the R.I. of the liquid phase is about the R.I. of the low refractive index abrasive silica.

The compositions of the present invention may furthermore contain optional ingredients, well-known in the art, in conventional amounts. Examples of such optional ingredients are binders such as sodium carboxymethylcellulose, and xanthan gum, buffer salts such as trisodium orthophosphate, solubilizing agents such as polyethylene glycols, surface-active agents such as anionic, nonionic and amphoteric synthetic surfactants, e.g. sodium laurylsulphate, flavours, sweetening agents, colouring agents, anticaries agents such as sodium fluoride and/or sodium monofluorophosphate, preservatives, etc.

Further optional ingredients are antibacterial agents, anti-plaque agents such as zinc citrate, stannous pyrophosphate, anti-calculus agents, plant extracts such as ziziphus juazeiro, bleaching agents such as percompounds, desensitising agents such as potassium salts, plaque buffers such as calcium glycerophosphate and urea etc.

Minor amounts of additional other abrasives may also be included, as long as the transparency of the gel is not jeopardized thereby, such as dicalcium phosphates, calciumpyrophosphates, calcium carbonates, and aluminas.

The compositions of the invention may be prepared as single compositions, or they may be prepared as multiple compositions, either for dispensing from a single container or from a multi compartment container. A preferred form is a dual composition, each composition having a different colour, said dual composition being dispensed from a single container.

The compositions of the invention may also contain coloured particles suspended therein, e.g. coloured silica agglomerates or other coloured particles to impart a "speckled" appearance to the dentifrices. The dentifrices may also contain stripes or a coloured paste, to provide for a visually clear gel-type dentifrice with coloured stripes or a separate coloured phase.

The present invention will be further illustrated by way of Example.

### Example 1

Clarity studies were carried out with the following formulation, in which the level of sodium bicarbonate was varied (1%, 5% and 10%).

Samples of the formulation were put in a cuvette, and the cuvette was placed on an illuminated platform, and a transparency scale ranging from -12 to +13 was placed behind the cuvette. The scale was slid behind the cuvette and a reading was taken when the characters on the scale became clearly readable.

The formulation used as follows:

| | % by weight |
|---|---|
| abrasive silica (according to EP 535943 and EP 666832) | 7 |
| abrasive silica (according to EP 236070) | 2 |
| thickening silica | 8 |
| sorbitol (70%) | 60 |
| sodium carboxymethylcellulose | 0.5 |
| trisodium orthophosphate | 0.1 |
| polyethylene glycol (MW 1500) | 5 |
| sodium laurylsulphate | 1.5 |
| flavour | 1.5 |
| saccharin | 0.1 |
| Pigment Blue | 0.01 |
| sodium monofluorophosphate | 0.6 |
| sodium bicarbonate | x |
| preservatives | 0.1 |
| water | to 100 |

The following results were obtained:

| | |
|---|---|
| x = 1% | Scale reading 0 to +4 |
| x = 5% | Scale reading -9 to -12 |
| x = 10% | Scale reading -9 to -12 |

Formulations based on Examples of present invention, but with additionally 1% titanium dioxide was tested for colour stability after storage for 1 month at 10, 28 and 40°C. The colour was measured by a standard Colour Stability Index Method, and the colour indices had the following meaning.
0 :- No colour change observed
1 :- Very slight colour change observed after a careful analysis
2 :- Slight colour change observed where a strong link with the original colour can be noticed
3 :- Moderate colour change where great part of the original colour is lost
4 :- Major colour change where most of the original colour is lost
5 :- Severe colour change with no link to the original one

The following results were obtained:
x = 1% Colour index : no yellowing after 1 month' storage at 10, 28 and 40°C
x = 10% Colour index : yellowing after 1 month' storage at 28°C and 40°C

### Example 2

The following are two-composition formulations according to the invention, to be dispensed from a single container, comprising 50% by weight of the white formulation and 50% by weight of the blue one.

| | % by weight | |
|---|---|---|
| | White formulation | Blue formulation |
| abrasive silica (according to EP 535943 and EP 666832) | 7-10 | 7-10 |
| abrasive silica (according to EP 236070) | 1-3 | 1-3 |
| thickening silica | 8-11 | 8-11 |
| sorbitol (70%) | 55-65 | 55-65 |
| sodium carboxymethylcellulose | 0.5-0.7 | 0.5-0.7 |
| trisodium orthophosphate | 0.1 | 0.1 |
| polyethylene glycol (MW 1500) | 3-5 | 3-5 |
| sodium laurylsulphate | 1.5-3 | 1.5-3 |
| flavour | 1-2 | 1-2 |
| saccharin | 0.1-0.3 | 0.1-0.3 |
| titanium dioxide | 1 | - |
| Pigment Blue | - | 0.01 |
| sodium monofluorophosphate | up to 1.2 | up to 1.2 |
| sodium bicarbonate | 0.5-5 | 0.5-5 |
| preservatives | up to 0.2 | up to 0.2 |
| water | to 100 | to 100 |

## Claims

1. An oral care composition in the form of a transparent gel, comprising a thickening silica, and an abrasive silica with a refractive index of 1.47 or below in a polyol-humectant-containing liquid vehicle,
**characterised in that** the gel further contains from 0.5 to 5% by weight of an alkalimetal bicarbonate source.

2. A composition according to claim 1, **characterised in that** the alkalimetal bicarbonate source is sodium bicarbonate.

3. A composition according to claims 1 or 2,
**characterised in that** it contains a colouring agent.

4. An oral care product for dispensing from a single or multicompartment container **characterised in that** it contains at least two compositions of different colour according to claims 1-3.

## Patentansprüche

1. Mundpflegezusammensetzung in Form eines transparenten Gels, umfassend ein verdickendes Siliziumdioxid und ein schleifendes Siliziumdioxid mit einem Brechungsindex von 1,47 oder darunter, in einem Polyol-Feuchthaltemittel-enthaltenden, flüssigen Träger, **dadurch gekennzeichnet, dass** das Gel weiterhin 0,5 bis 5 Gewichtsprozent einer Alkalimetallbicarbonatquelle enthält.

2. Zusammensetzung nach Anspruch 1, **dadurch gekennzeichnet, dass** die Alkalimetallbicarbonatquelle Natriumbicarbonat ist.

3. Zusammensetzung nach Ansprüchen 1 oder 2, **dadurch gekennzeichnet, dass** sie ein färbendes Mittel enthält.

4. Mundpflegeprodukt zur Ausgabe aus einem Ein- oder Mehrkammerbehälter, **dadurch gekennzeichnet, dass** es mindestens zwei Zusammensetzungen von unterschiedlicher Farbe nach Ansprüchen 1-3 enthält.

## Revendications

1. Composition d'hygiène buccale sous la forme d'un gel transparent comprenant une silice épaississante et une silice abrasive ayant un indice de réfraction de 1,47 ou inférieur dans un matériau formant support liquide contenant de l'humectant polyol, **caractérisé en ce que** le gel contient en outre de 0,5 à 5 % en poids d'une source de bicarbonate de métal alcalin.

2. Composition selon la revendication 1, **caractérisé en ce que** la source de bicarbonate de métal alcalin est un bicarbonate de sodium.

3. Composition selon la revendication 1 ou 2, **caractérisée en ce qu'**elle contient un agent colorant.

4. Produit d'hygiène buccale destiné à être distribué à partir d'un récipient à un seul compartiment ou à plusieurs compartiments, **caractérisé en ce qu'**il contient au moins deux compositions de couleurs différentes selon les revendications 1 - 3.
